# EUROPEAN PATENT APPLICATION

(11) **EP 4 349 376 A1**
(43) Date of publication of application: **10.04.2024**
(21) Application number: 22811415.3
(22) Date of filing: 27.05.2022
(51) Int. Cl.: A61L 27/36, A61K 39/395, A61K 45/06, A61L 27/38, A61P 1/04, A61P 7/00, A61P 7/02, A61P 31/04, A61P 31/12, A61P 37/06

(54) **TRANSPLANT MATERIAL AND KIDNEY TRANSPLANT KIT**

(30) Priority: 28.05.2021 JP 2021090066
(71) Applicant: The Jikei University, Tokyo 105-8461 (JP); Sumitomo Pharma Co., Ltd., Osaka 541-0045 (JP); BIOS Co., Ltd, Tokyo 110-0015 (JP)
(72) Inventor: KOBAYASHI, Eiji, Tokyo 105-8461 (JP); YOKOO, Takashi, Tokyo 105-8461 (JP); KIYOSHI, Akihiko, Suita-shi, Osaka 564-0053 (JP); KURODA, Takao, Kobe-shi, Hyogo 650-0047 (JP)
(74) Representative: V.O.
(86) International application number: PCT/JP2022/021782
(87) International publication number: WO 2022/250147

(57) **Abstract**

A transplant material used for kidney xenotransplantation to primates is provided, the transplant material comprising a kidney anlage with bladder derived from a pig fetus devoid of blood vessels.

## Description

### [Technical Field]

The present invention relates to a transplant material and a kidney transplant kit.

Priority is claimed on Japanese Patent Application No. 2021-090066 filed May 28, 2021, the content of which is incorporated herein by reference.

### [Background Art]

Since organ transplantation was first performed, xenogeneic donor organs have attracted attention as means for avoiding the problem of donor shortage. Particularly, with the advance of developmental biotechnology for pigs, genetically engineered pigs useful for xenotransplantation have been produced and are in the process of being tested in preclinical models using non-human primates as recipients (see Non-Patent Document 1).

In recent years, genetically modified pigs have been developed, and there have been reports on the long-term survival achieved by control with extremely potent immunosuppressants (see Non-Patent Documents 2 to 4).

### [Citation List]

### [Non-Patent Documents]

### [Non-Patent Document 1]

Qi S, Peng J, Xu D, Vu MD, Liu D, Chen H. improved techniques for kidney transplantation in the monkey. Microsurgery. 1999; 19(7): 335-7.

### [Non-Patent Document 2]

Iwase H, Hara H, Ezzelarab M, et al. Immunologic and physiologic observations in baboons with life-supporting genetically engineered pig kidney grafts. Xenotransplantation. 2017; 24(2): 10.1111/xen.12293.

### [Non-Patent Document 3]

Higginbotham L, Mathews D, Breeden CA, et al. Pre-transplant antibody screening and anti-CD 154 costimulation blockade promote long-term xenograft survival in a pig-to-primate kidney transplant model. Xenotransplantation. 2015; 22(3): 221 -230. doi: 10.1111/xen.12166

### [Non-Patent Document 4]

Kim SC, Mathews DV, Breeden CP, et al. Long-term survival of pig-to-rhesus macaque renal xenografts is dependent on CD4 T cell depletion. Am J Transplant. 2019; 19(8): 2174-2185. doi: 10.1111/ajt.15329

### [Summary of Invention]

### [Technical Problem]

However, in the pig-to-primate kidney xenotransplantation, controlling immune responses with only clinically approved immunosuppressants is extremely difficult and needs to be further improved.

An object of the present invention is to provide a transplant material and a kidney transplant kit that are capable of suppressing the rejection reaction of a recipient when being transplanted into primates and accomplishing long-term survival.

### [Solution to Problem]

The present invention includes the following aspects.
[1] A transplant material used for kidney xenotransplantation to primates, the transplant material comprising a kidney anlage with bladder derived from a pig fetus devoid of blood vessels.
[2] The transplant material described in [1], in which the kidney anlage with bladder is derived from a pig fetus in week 4 to 6 of an embryonic period.
[3] The transplant material described in [1] or [2], in which the kidney anlage with bladder is derived from a wild-type pig fetus.
[4] The transplant material described in any one of [1] to [3], in which the kidney anlage with bladder further comprises human-derived nephron progenitor cells.
[5] The transplant material described in any one of [1] to [4], in which the transplant material is used for a patient receiving immunosuppressants based on an immunosuppressive protocol.
[6] The transplant material described in [5], in which the immunosuppressive protocol includes
   (1) administering at least one immunosuppressant selected from thymoglobulin and an anti-CD20 antibody, and
   (2) administering at least one immunosuppressant selected from a CD28-mediated co-stimulatory signal inhibitor, a calcineurin inhibitor, an inosine monophosphate dehydrogenase inhibitor, a steroid, an anti-CD25 antibody, and an anti-CD40 antibody.
[7] The transplant material described in [6], in which the immunosuppressive protocol further includes
   (3) administering at least one anti-inflammatory agent selected from an anti-IL-6 receptor antibody and a TNF-α inhibitor, and/or
   (4) administering at least one adjunctive agent selected from a platelet aggregation inhibitor, an anticoagulant, an antibiotic, a hematopoietic agent, a gastric acid suppressant, and an antiviral agent.
[8] The transplant material described in any one of [5] to [7], in which the immunosuppressive protocol includes
   (1) independently administering thymoglobulin and an anti-CD20 antibody 2 or 3 days before transplantation,
   (2) administering a CD28-mediated co-stimulatory signal inhibitor every 7 days after the transplantation, or immediately after the transplantation and every 7 days,
   (3) continuously administering a calcineurin inhibitor from 9 days before the transplantation,
   (4) continuously administering inosine monophosphate dehydrogenase inhibitor from 5 days before the transplantation,
   (5) continuously administering a steroid from the day of the transplantation,
   (6) administering an anti-IL-6 receptor antibody 1 to 3 times on the day before the transplantation and about every 7 days after the transplantation,
   (7) administering a TNF-α inhibitor every 3 or 4 days immediately after the transplantation, and
   (8) administering at least one agent selected from a platelet aggregation inhibitor, an anticoagulant, an antibiotic, a hematopoietic agent, a gastric acid suppressant, and an antiviral agent, as an adjunctive agent.
[9] The transplant material described in any one of [5] to [7], in which the immunosuppressive protocol includes
   (1) administering an anti-CD20 antibody from about 2 to 3 weeks before transplantation,
   (2) continuously administering an inosine monophosphate dehydrogenase inhibitor from about 2 weeks before the transplantation,
   (3) administering an anti-CD25 antibody on the day of the transplantation and 4^{th} day after the transplantation,
   (4) continuously administering a calcineurin inhibitor and a steroid from about 1 week before the transplantation; and
   (5) administering at least one agent selected from a platelet aggregation inhibitor, an anticoagulant, an antibiotic, a hematopoietic agent, a gastric acid suppressant, and an antiviral agent, as an adjunctive agent.
[10] A kidney transplant kit comprising the transplant material described in any one of [1] to [4], and a combination of drugs used based on an immunosuppressive protocol.
[11] The kidney transplant kit described in [10], in which the immunosuppressive protocol includes
   (1) administering at least one immunosuppressant selected from thymoglobulin and an anti-CD20 antibody, and
   (2) administering at least one immunosuppressant selected from a CD28-mediated co-stimulatory signal inhibitor, a calcineurin inhibitor, an inosine monophosphate dehydrogenase inhibitor, a steroid, an anti-CD25 antibody, and an anti-CD40 antibody.
[12] The kidney transplant kit described in [11] in which the immunosuppressive protocol further includes
   (3) administering at least one anti-inflammatory agent selected from an anti-IL-6 receptor antibody and a TNF-α inhibitor, and/or
   (4) administering at least one adjunctive agent selected from a platelet aggregation inhibitor, an anticoagulant, an antibiotic, a hematopoietic agent, a gastric acid suppressant, and an antiviral agent.
[13] The kidney transplant kit described in any one of [10] to [12], in which the immunosuppressive protocol includes
   (1) independently administering thymoglobulin and an anti-CD20 antibody 2 or 3 days before transplantation,
   (2) administering a CD28-mediated co-stimulatory signal inhibitor every 7 days after the transplantation, or immediately after the transplantation and every 7 days,
   (3) continuously administering a calcineurin inhibitor from 9 days before the transplantation,
   (4) continuously administering inosine monophosphate dehydrogenase inhibitor from 5 days before the transplantation,
   (5) continuously administering a steroid from the day of the transplantation,
   (6) administering an anti-IL-6 receptor antibody 1 to 3 times on the day before the transplantation and about every 7 days after the transplantation,
   (7) administering a TNF-α inhibitor every 3 or 4 days immediately after the transplantation, and
   (8) administering a platelet aggregation inhibitor, an anticoagulant, an antibiotic, a hematopoietic agent, a gastric acid suppressant, and an antiviral agent as adjunctive agents.
[14] The kidney transplant kit described in any one of [10] to [12], in which the immunosuppressive protocol includes
   (1) administering an anti-CD20 antibody from about 2 to 3 weeks before transplantation,
   (2) continuously administering an inosine monophosphate dehydrogenase inhibitor from about 2 weeks before the transplantation,
   (3) administering an anti-CD25 antibody on the day of the transplantation and 4^{th} day after the transplantation,
   (4) continuously administering a calcineurin inhibitor and a steroid from about 1 week before the transplantation; and
   (5) administering at least one agent selected from a platelet aggregation inhibitor, an anticoagulant, an antibiotic, a hematopoietic agent, a gastric acid suppressant, and an antiviral agent, as an adjunctive agent.

### [Advantageous Effects of Invention]

According to the present invention, it is possible to provide a transplant material and a kidney transplant kit that are capable of suppressing the rejection reaction of a recipient when being transplanted into primates and accomplishing long-term survival.

### [Brief Description of Drawings]

FIG. 1 shows blood test data of each animal in examples. (A) No. 200 (Experiment 1), (B) No. 335 (Experiment 1), (C) No. 323 (Experiment 2), and (D) No. 219 (Experiment 2).
FIG. 2 shows photographs of a neonatal kidney transplanted in Experiment 1. (A) the day of transplantation and (B) 13 days after transplantation.
FIG. 3 shows photographs of an embryonic cloaca transplanted in Experiment 1. (A) 13 days after transplantation and (B) 27 days after transplantation.
FIG. 4 shows typical photographs comparing the histopathological characteristics of the neonatal kidney and the embryonic cloaca in Experiment 1. (A) to (C): neonatal kidney, (D) to (F): embryonic cloaca (13 days after transplantation), (G) to (I): embryonic cloaca (27 days after transplantation), (A), (B), (D), (E), (G), and (H): Masson's trichrome staining images, and (C), (F), and (I): CD3.
FIG. 5 shows photographs of an embryonic cloaca transplanted in Experiment 2. (A) 33 days after transplantation, (B) 50 days after transplantation, and (C) 79 days after transplantation.
FIG. 6 shows typical photographs comparing histopathological characteristics of the embryonic cloaca in Experiment 2. (A) to (C): 33 days after transplantation, (D) to (F): 50 days after transplantation, (G) to (I): 79 days after transplantation, (A), (B), (D), (E), (G), and (H): Masson's trichrome staining images, and (C), (F), and (I): CD.3

### [Description of Embodiments]

### «Transplant material»

In one embodiment, the present invention provides a transplant material used for kidney xenotransplantation to primates, the transplant material comprising a kidney anlage with bladder derived from a pig fetus devoid of blood vessels.

The transplant material of the present embodiment is suitably used for improving the renal function of primates with renal dysfunction.

### <<Kidney anlage>>

The kidney anlage refers to the kidney in the embryonic stage, which corresponds to the metanephroi in mammals. In mammals at the embryonic stage, a tissue where bladder-ureter-metanephric tissue is integrated is formed. In the present specification, such a tissue is called a kidney anlage with bladder. The inventors of the present invention named the bladder-ureter-metanephric tissue at the embryonic stage "cloaca graft". That is, in the present specification, the kidney anlage with bladder is also called a cloaca graft. In a case where the kidney anlage is transplanted into the abdominal cavity of an animal, blood vessels invade the cloaca from the recipient, and the cloaca keeps growing and produces urine.

As will be described later in Examples, as a result of transplanting each of a neonatal pig kidney with blood vessels and a kidney anlage with bladder derived from a pig fetus devoid of blood vessels to cynomolgus monkeys as recipients, in the pig-to-monkey kidney xenotransplantation, the immunogenicity of the embryonic kidney anlage (pig fetus-derived kidney anlage with bladder) was lower than that of the neonatal kidney.

In a neonatal kidney graft accompanying vascular anastomosis, an acute rejection reaction was observed after several weeks. On the other hand, although the same immunosuppressive protocol was implemented, the kidney anlage of a pig fetus (pig fetus-derived kidney anlage with bladder) avoided a rejection reaction and kept developing for a long period of time, specifically, at least about 80 days.

That is, the inflow blood vessels of the developing (or growing) kidney after transplantation were of the recipient, which showed that the initial target of a rejection reaction against a different species, that is, vascular endothelial dysfunction as one of the hyperacute rejection reactions can be avoided using the kidney anlage with bladder derived from a pig fetus devoid of blood vessels.

The kidney anlage with bladder is preferably derived from a pig fetus in week 4 to 6 of the embryonic period (28 to 42 days), and is more preferably derived from a pig fetus on day 28 to 30 of the embryonic period. Using such an embryonic kidney makes it easy for the tissue to mature into urine-producing renal tissue without differentiating into other tissues.

Furthermore, the blood vessels of a xenogeneic recipient flow into the kidney of the fetus in the process of transplantation, which is likely to suppress the expression of the donor antigen in the development stage of the kidney.

In one aspect, the kidney anlage with bladder is preferably derived from a wild-type pig fetus. That is, in the transplantation of pig-derived renal tissue to primates in the related art, pig genes have to be modified to avoid a strong rejection reaction of the recipient; however, in the present invention, as will be described later in Examples, it has been confirmed that using the kidney anlage with bladder derived from a wild-type pig fetus that does not accompany genetic modification as a transplant material suppresses an acute rejection reaction.

In one aspect, it is preferable that the genetic modification for avoiding a rejection reaction in xenotransplantation not be performed on the kidney anlage with bladder. Examples of the genetic modification include methods known to those skilled in the art (for example, Xiao-Hua Yu et al., Clinica Chimica Acta 514 (2021) 15-23, or Lu et al., Frontiers in Immunology, January 2020, Volume 10, Article 3060). Specifically, examples thereof include α1,3-galactosyltransferase (GalT) knockout, CMP-N-acetylneuraminic acid hydroxylase (CMAH) knockout, β-1,4N-acetylgalactosaminyltransferase (β4GalNT2) knockout, human CD39 (hCD39) gene introduction, human CD46 (hCD46) gene introduction, human CD47 (hCD47) gene introduction, human CD55 (hCD55) gene introduction, human CD59 (hCD59) gene introduction, human CD200 (hCD200) gene introduction, human CD274 (hCD274) gene introduction, human thrombomodulin (hTBM) gene introduction, human tissue factor pathway inhibitor (hTFPI) gene introduction, human endothelial protein C receptor (hEPCR) gene introduction, human tumor necrosis factor alpha-induced protein-3 (hA20) gene introduction, human heme oxygenase 1 (hHO1) gene introduction, cytotoxic T-lymphocyte-associated protein-4-immunoglobulin (CTLA4-Ig) gene introduction, and MHC class II transactivator dominant negative (CIITA-DN) gene introduction.

The recipient of xenotransplantation of the transplant material of the present embodiment is primates. Examples of the primates include humans, monkeys, gorillas, chimpanzees, and the like. Examples of the monkeys include rhesus monkeys, cynomolgus monkeys, and the like. Examples of one aspect of the primates include old world monkeys such as cynomolgus monkeys or humans. Examples of one aspect of the primates include primates that do not express galactose-α-1,3-galactose.

From the viewpoint of establishing a urine excretion system, the transplant material of the present embodiment is preferably used such that the bladder of the kidney anlage with bladder is transplanted and connected to the recipient's ureter.

The kidney anlage with bladder may further comprise human-derived nephron progenitor cells. Examples of the nephron progenitor cells include cells obtained by inducing differentiation from pluripotent stem cells such as iPS cells and ES cells. The nephron progenitor cells may be cells derived from a patient or may be allogeneic cells treated to suppress a rejection reaction (see Fujimoto T, Yamanaka S, Tajiri S, et al. Generation of Human Renal Vesicles in Mouse Organ Niche Using Nephron Progenitor Cell Replacement System. Cell Rep. 2020; 32 (11): 108130. doi: 10.1016/j. celrep. 2020. Article No. 108130).

The human-derived nephron progenitor cells are injected into the kidney anlage of a pig fetus. Using the kidney anlage with bladder further comprising the human-derived nephron progenitor cells allows a chimeric embryonic kidney to grow in the recipient.

The size of organ in the transplant material is preferably the same as the size of organ that the recipient originally had. Therefore, for example, in order to form a kidney that can fully function in a human, it is preferable that the donor be a mammal having an organ size similar to that of a human. However, it is not necessary for the organ sizes to be exactly identical. In the case of the kidney, as long as the kidney has one tenth of the whole function, the kidney can sufficiently perform dialysis and sufficiently maintain life. For this reason, a pig is used as a donor of the transplant material of the present embodiment, and the organ size of a miniature pig is considered to be sufficient.

Next, the kidney anlage with bladder prepared as described above is transplanted into the abdominal cavity of a recipient. The transplantation site is not limited as long as it is in the abdominal cavity, and examples thereof include the greater omentum, the paraaortic region, and the like. The greater omentum is a peritoneum that hangs down like an apron from the lower side of the stomach.

The transplantation of the kidney anlage to the greater omentum, the paraaortic region, and the like of primates including humans can be performed by a conventional surgical procedure or the like. Examples thereof include a method of picking up the kidney anlage to be transplanted with sharp tweezers, making a small incision on the surface of adipose tissue of the greater omentum with the tip of the tweezers, and embedding the tissue in the greater omentum. Furthermore, an endoscope can also be used to transplant the kidney anlage into the greater omentum, the paraaortic region, and the like.

### <<Kidney transplant kit>>

In one embodiment, the present invention provides a transplant method using the aforementioned transplant material based on an immunosuppressive protocol, that is, the transplant material used based on an immunosuppressive protocol.

Furthermore, in one embodiment, the present invention provides a kidney transplant kit including the aforementioned transplant material and a combination of drugs used based on an immunosuppressive protocol.

Examples of the combination of drugs used based on the immunosuppressive protocol include a combination of an immunosuppressant and an anti-inflammatory agent and/or an adjunctive agent.

In the present specification, an immunosuppressant means an agent that suppresses the generation, activation, or proliferation of immune cells. Specific examples of the immune cells include lymphocytes such as B cells and T cells. Examples of the immunosuppressant include a drug that damages T cells, a drug that inhibits T cell synthesis, a drug that suppresses T cell activation, a drug that suppresses differentiation (synthesis)/proliferation of T cells or activation/proliferation of B cells, and the like. In addition, a drug that indirectly suppresses the generation, activation, or proliferation of immune cells, or a drug such as an anti-inflammatory agent that suppresses the secretion or activity of cytokine caused by the action of immune cells is also in the category of the immunosuppressant.

Specific examples of the drug that damages T cells or B cells include thymoglobulin that damages T cells, an anti-CD20 antibody that damages B cells (for example, rituximab and obinutuzumab), and the like.

Specific examples of the drug that suppresses the differentiation/activation or proliferation of T cells include a substance that inhibits the CD28 co-stimulatory signal (for example, abatacept including a CTLA4 extracellular domain that binds to CD80/CD86), a calcineurin inhibitor (for example, tacrolimus and ciclosporin), an inosine monophosphate dehydrogenase inhibitor (for example, mycophenolic acid, mycophenolate mofetil (MMF), which is an ester of mycophenolic acid, azathioprine, and mizoribine), a steroid (for example, methylprednisolone, which is a glucocorticoid-based steroid), an anti-CD25 antibody (for example, basiliximab), an anti-CD40 antibody (for example, iscalimab (CFZ533)), and the like.

Examples of the anti-inflammatory agent include an IL-6 inhibitor (for example, tocilizumab or sarilumab, which is an anti-IL-6 receptor antibody), a TNFα inhibitor (for example, etanercept, which is a soluble TNFα receptor, or adalimumab, golimumab, or certolizumab pegol, which is an anti-TNFα monoclonal antibody), and the like.

Examples of the adjunctive agent include a platelet aggregation inhibitor (such as acetylsalicylic acid (aspirin)), a gastric acid suppressant (such as famotidine), an anticoagulant (such as low-molecular-weight heparin or nafamostat), an antibiotic (for example, a penicillin-based antibiotic, a cephem-based antibiotic, a sulfa agent, or the like), a hematopoietic agent (such as erythropoietin or roxadustat), an antiviral agent (such as valganciclovir or ganciclovir), and the like.

The method of administering these drugs is not particularly limited, and may be appropriately determined based on the immunosuppressive protocol. For example, a tablet, a coated tablet, a pill, powder, granules, a capsule, a liquid, a suspension, an emulsion, or the like is administered orally. In addition, an intravenous injection is administered singly or as a mixture with a general replacement fluid such as glucose or amino acids. Furthermore, as necessary, an intraarterial, intramuscular, intradermal, subcutaneous, or intraperitoneal injection is administered. Suppositories are administered rectally. A topical agent is applied, affixed, or sprayed onto an affected area.

The dose of these drugs cannot be determined uniformly because it varies with symptoms, body weight, age, gender, and the like, of a patient. In the case of oral administration, for example, a daily dose thereof may be 1 µg to 10 g. For instance, a daily dose of the active ingredient may be 0.01 to 2,000 mg. In the case of injection, for example, a daily dose thereof may be 0.1 µg to 1 g. For instance, a daily dose of the active ingredient may be 0.001 to 200 mg. In the case of a suppository, for example, a daily dose thereof may be 1 µg to 10 g. For instance, a daily dose of the active ingredient may be 0.01 to 2,000 mg.

There is no particular limitation on the immunosuppressive protocol in the present specification, that is, the combination of immunosuppressants and the timing of administration. Examples thereof include
(1) administering a relatively potent immunosuppressant, for example, a drug that damages immune cells at the induction phase of immunosuppression, and
(2) administering for a certain period of time or more an immunosuppressant, a drug that inhibits the differentiation (synthesis), activation, or proliferation of immune cells, and/or an anti-inflammatory agent having low safety concern with prolonged administration at the maintenance phase of immunosuppression.

In the induction phase, for example, at any point during a period from about 2 weeks before transplantation to several days after transplantation (for instance, the day of transplantation to 4^{th} day after the transplantation), the drug can be administered once or multiple times.

In the maintenance phase, for example, during a period that lasts for a certain period of time after transplantation from about 2 weeks before transplantation, the drugs can be continuously administered (for instance, once a day, once every 2 days, or the like).

Examples of the immunosuppressive protocol in the present embodiment include a protocol including
(1) administering at least one immunosuppressant selected from thymoglobulin and an anti-CD20 antibody, and
(2) administering at least one immunosuppressant selected from a CD28-mediated co-stimulatory signal inhibitor (for example, abatacept), a calcineurin inhibitor (for example, tacrolimus or rapamycin), an inosine monophosphate dehydrogenase inhibitor (for example, mycophenolate mofetil (MMF)), a steroid (for example, methylprednisolone), an anti-CD25 antibody, and an anti-CD40 antibody.

As one aspect, in (2), it is preferable to administer at least one immunosuppressant selected from a CD28-mediated co-stimulatory signal inhibitor (for example, abatacept), a calcineurin inhibitor (for example, tacrolimus or rapamycin), an inosine monophosphate dehydrogenase inhibitor (for example, mycophenolate mofetil (MMF)), a steroid (for example, methylprednisolone), and an anti-CD25 antibody.

Such a protocol may further include
(3) administering at least one anti-inflammatory agent selected from an anti-IL-6 receptor antibody and a TNF-α inhibitor, and/or
(4) administering at least one adjunctive agent selected from a platelet aggregation inhibitor, an anticoagulant, an antibiotic, a hematopoietic agent, a gastric acid suppressant, and an antiviral agent.

Examples of one aspect of the immunosuppressive protocol include a protocol including
(1) independently administering thymoglobulin and an anti-CD20 antibody 2 or 3 days before transplantation,
(2) administering a CD28-mediated co-stimulatory signal inhibitor every 7 days after the transplantation, or after the transplantation and every 7 days,
(3) continuously administering a calcineurin inhibitor from 9 days before the transplantation,
(4) continuously administering inosine monophosphate dehydrogenase inhibitor from 5 days before the transplantation,
(5) continuously administering a steroid from the day of the transplantation,
(6) administering an anti-IL-6 receptor antibody 1 to 3 times on the day before the transplantation and about every 7 days after the transplantation, specifically, on the 7th day and 14th day after the transplantation,
(7) administering a TNFα inhibitor (for example, etanercept) every 3 or 4 days immediately after the transplantation, and
(8) administering a platelet aggregation inhibitor, an anticoagulant, an antibiotic, a hematopoietic agent, a gastric acid suppressant, and an antiviral agent as adjunctive agents.

Examples of specific protocols include a protocol including
(1) independently administering thymoglobulin and rituximab 2 or 3 days before transplantation,
(2) administering abatacept every 7 days after the transplantation, or after the transplantation and every 7 days,
(3) continuously administering tacrolimus from 9 days before the transplantation,
(4) continuously administering MMF from 5 days before the transplantation,
(5) continuously administering methylprednisolone from the day of the transplantation,
(6) administering tocilizumab on the day before the transplantation and on the 7th day and 14th day after the transplantation,
(7) administering etanercept every 3 or 4 days immediately after the transplantation, and
(8) administering at least one agent selected from aspirin, low-molecular-weight heparin, an antibiotic, erythropoietin, and valganciclovir as an adjunctive agent.

Examples of one aspect of the immunosuppressive protocol include a protocol including
(1) administering an anti-CD20 antibody from about 2 to 3 weeks before transplantation,
(2) continuously administering an inosine monophosphate dehydrogenase inhibitor from about 2 weeks before the transplantation,
(3) administering an anti-CD25 antibody on the day of the transplantation and 4^{th} day after the transplantation,
(4) continuously administering a calcineurin inhibitor and a steroid from about 1 week before the transplantation; and
(5) administering a platelet aggregation inhibitor, an anticoagulant, an antibiotic, a hematopoietic agent, a gastric acid suppressant, and an antiviral agent as adjunctive agents.

Examples of specific protocols include a protocol including
(1) administering rituximab from about 2 to 3 weeks before transplantation,
(2) continuously administering MMF from about 2 weeks before the transplantation,
(3) administering basiliximab on the day of the transplantation and 4^{th} day after the transplantation,
(4) continuously administering tacrolimus and methylprednisolone from about 1 week before the transplantation, and
(5) administering at least one agent selected from a platelet aggregation inhibitor, the anticoagulant, the antibiotic, the hematopoietic agent, the gastric acid suppressant, and the antiviral agent, as an adjunctive agent.

### «Transplant method»

In one embodiment, the present invention provides a kidney transplant method including a step (a) of transplanting a transplant material comprising a kidney anlage with bladder derived from a pig fetus devoid of blood vessels into the body of a patient, and a step (b) of connecting the bladder derived from the pig fetus to the ureter of the patient after a predetermined period following transplantation.

It can be said that the method of the present embodiment is a method of treating kidney diseases. As the transplant material, it is possible to use the transplant material described above.

In a case where the transplant material transplanted in the above step (a) is left as it is for a predetermined period, the transplant material grows and begins to produce urine. The predetermined period may be a period that lasts until the transplant material fully grows but has not caused hydronephrosis yet. The predetermined period may be appropriately adjusted depending on the patient's symptoms and the like. For example, the predetermined period may be 1 to 10 weeks.

Subsequently, step (b) is carried out after the predetermined period following the transplantation. In step (b), the bladder of the transplant material is cut, and the bladder and the patient's ureter are connected. Examples of the connection method include end-to-end anastomosis, end-to-side anastomosis, side-to-side anastomosis, and side-to-end anastomosis. Among these, end-to-side anastomosis is preferable.

Connecting the bladder of the transplant material and the patient's bladder via the patient's ureter enables the urine produced by the transplant material to be excreted to the patient's bladder. More specifically, the urine produced by the kidney of the transplant material is excreted to the bladder of the transplant material, and then excreted from the bladder of the transplant material to the patient's bladder through the patient's ureter.

That is, examples of one embodiment of the present invention include a transplant material comprising an embryonic pig kidney anlage with bladder used in the aforementioned kidney transplant method or the aforementioned method of treating kidney diseases.

Furthermore, examples of one embodiment of the present invention include use of an embryonic pig kidney anlage with bladder for manufacturing a transplant material, the kidney anlage with bladder being used in the aforementioned kidney transplant method or in the aforementioned method of treating kidney diseases.

It is preferable that the transplant method be carried out together with the implementation of the immunosuppressive protocol described above. The immunosuppressive protocol may be any of the immunosuppressive protocols described in the present specification, such as [6] to [9] and [11] to [14] described above.

In one embodiment, examples of the present invention include a transplant material comprising an embryonic pig kidney anlage with bladder used in the aforementioned kidney transplant method or the aforementioned method of treating kidney diseases carried out together with the implementation of an immunosuppressive protocol, and a kidney transplant kit including a combination of drugs used based on the transplant material and the immunosuppressive protocol.

Furthermore, examples of one embodiment of the present invention include a transplant material comprising an embryonic pig kidney anlage with bladder used in the aforementioned kidney transplant method or the aforementioned method of treating kidney diseases carried out together with the implementation of an immunosuppressive protocol, and use of the transplant material for manufacturing a kidney transplant kit including a combination of drugs used based on the transplant material and the immunosuppressive protocol. For the purpose of suppressing a rejection reaction of a recipient (patient) against the transplant material, the transplant method can be performed after plasma exchange is performed for the recipient.

In one embodiment, the present invention provides a kidney transplant method or a method of treating kidney diseases, including a step (a1) of performing plasma exchange for a recipient (patient), a step (b1) of transplanting a transplant material comprising a kidney anlage with bladder derived from a pig fetus that is devoid of blood vessels into the patient's body, and a step (b2) of connecting the bladder derived from the pig fetus to the patient's ureter after a predetermined period following transplantation.

That is, examples of one embodiment of the present invention include a transplant material comprising an embryonic pig kidney anlage with bladder used in the aforementioned kidney transplant method or the aforementioned method of treating kidney diseases.

Furthermore, examples of one embodiment of the present invention include use of an embryonic pig kidney anlage with bladder for manufacturing a transplant material, the kidney anlage with bladder being used in the aforementioned kidney transplant method or in the aforementioned method of treating kidney diseases.

As the method of plasma exchange, double filtration plasmapheresis (DFPP), simple plasma exchange (PE), plasma adsorption (PA), immunoadsorption plasmapheresis (IAPP), and the like are known. It is preferable that the plasma exchange before transplantation and the implementation of the aforementioned immunosuppressive protocol be performed together.

### [Examples]

Hereinafter, the present invention will be described based on examples, but the present invention is not limited to the following examples.

### [Abbreviations]

TAC: Tacrolimus
MMF: Mycophenolate mofetil

### [Experiment 1]

A wild pig was used as a donor, and a cryopreserved cloaca thereof on day 28 to 30 of the embryonic period was thawed and used as an embryonic kidney. In addition, the kidney of a 20- to 28-day-old neonatal pig weighing 5.0 to 6.12 kg was also used as a donor kidney. Experiments were carried out in accordance with the appropriate guidelines for animal experiments.

### [Transplantation of neonatal pig kidney and embryonic cloaca]

The transplantation of neonatal pig kidney accompanying vascular anastomosis was performed according to the method described in Non-Patent Document 2. The renal blood flow from the neonatal pig kidney donor was blocked after midline abdominal incision.

Subsequently, the blood flow was restored in a preservation solution containing 465 mL of Euro-Collins solution, 35 mL of a 50% glucose solution, and 1,000 units of heparin, the kidney was removed along with the blood vessel and ureter, and the kidney was transported to recipient facilities to prepare for transplantation (total time of preservation, 3 hours).

All recipient monkeys were fasted from the evening before surgery until the time of surgery. For maintenance of anesthesia, atropine (0.1 mg/kg i.m.) and ketamine (10 mg/kg i.m.) were introduced on the day of surgery, and then isoflurane (0.5% to 2%) was administered by inhalation.

Furthermore, butorphanol (0.1 mg/kg i.m.) was used as an analgesic, and benzylpenicillin potassium was used at 47,500 units/individual as an antibiotic before and after the surgery.

Under general anesthesia, laparotomy was performed on the aforementioned pretreated recipient monkeys through midline abdominal incision. The left renal artery/vein and the left ureter were ligated, the left kidney was then extracted, the neonatal pig kidney was placed where the left kidney was, and the blood vessel and ureter were connected by anastomosis.

Next, the posterior peritoneum near the aorta and the left kidney was removed by blunt dissection to form a pocket, and two embryonic cloacae (cloaca grafts (kidney anlages with bladder)) were transplanted to the pocket of each animal by using a spatula. After transplantation, the pocket was closed with LIGACLIP (registered trademark). In the same way, two embryonic cloacae were transplanted to the greater omentum, and the pocket was closed with LIGACLIP (registered trademark). By being covered with the tissue of the greater omentum in this way, the cloacae were prevented from detaching. After the cloacae were transplanted to the periphery of the aorta and the greater omentum, the abdomen and wound were closed to finish the surgery.

### [Recovery of transplanted tissue]

Thirteen days after transplantation, the abdomen was opened again by midline abdominal incision. After the abdomen was opened, the greater omentum was exposed to identify the kidneys derived from the transplanted embryonic cloacae by using the portion clipped with LIGACLIP (registered trademark) as a guide. A part of the kidneys derived from the transplanted embryonic cloacae was dissected together with the surrounding greater omentum tissue to avoid damage, and the tissue was recovered.

Subsequently, the intestine was manually moved to expose the transplanted kidney derived from the neonatal pig. The surroundings of the left renal artery/vein and the left ureter were removed to expose the abdominal aorta and the inferior vena cava. The left renal vein and the left ureter were ligated, and the transplanted kidney derived from the neonatal pig was cut on the side of the pig's renal artery and vein and extracted as a whole. Finally, the abdomen and the wound were closed to finish the surgery. The administration of immunosuppressants was continued even after the surgery under the monitoring of the dietary intake status.

Furthermore, 27 days after the transplantation, the remaining kidneys derived from the embryonic cloacae transplanted to the greater omentum were identified again by using the same method, and the transplanted tissue was recovered.

### [Experiment 2]

### [Embryonic kidney transplantation]

The pre- and post-operative preparations for the recipient monkey were carried out substantially in the same manner as in Experimental Example 1. The entire abdomen was sterilized with isodine, an incision was made at the center, and the abdomen was opened after administration of an anesthetic. The greater omentum and the intestine were manually moved to expose the posterior peritoneum after opening of the abdomen. The posterior peritoneum near the aorta and the left kidney was removed by blunt dissection to form a pocket, and two embryonic cloacae were transplanted to the pocket of each animal by using a spatula. After the transplantation, the pocket was closed with LIGACLIP (registered trademark). Subsequently, three embryonic cloacae were transplanted to the greater omentum in the same manner as described above, and the pocket was closed with LIGACLIP (registered trademark). In this way, the embryonic cloacae were covered with the greater omentum tissue so as not to be detached. After the embryonic cloacae were transplanted to the periphery of the aorta and the greater omentum, the abdomen and the wound were closed to finish the surgery.

### [Recovery of embryonic kidney in greater omentum/embryonic bladder around aorta and urinary tract anastomosis]

Thirty-three days after the transplantation, the abdomen was opened again by midline abdominal incision. After the abdomen was opened, the greater omentum was exposed, and the entire kidney derived from the transplanted embryonic cloacae was identified using the portion clipped with LIGACLIP (registered trademark) as a guide. Next, the kidney derived from the embryonic cloacae was dissected together with the surrounding greater omentum tissue to avoid damage, and the tissue was recovered.

Likewise, the kidney derived from the embryonic cloacae transplanted to the paraaortic region was identified as well, the region surrounding the left renal artery/vein and the left ureter was removed to expose the adjacent abdominal aorta and inferior vena cava. After the left renal artery, the left renal vein and the left ureter were ligated, and the left kidney was extracted. After the removal, one embryonic bladder and the left ureter of the recipient monkey were connected in the manner of end-to-end anastomosis by parachute suturing for urinary reconstruction. Finally, after the urinary reconstruction, the abdomen and the wound were closed to finish the surgery.

### [Recovery of paraaortic embryonic kidney after ureteral reconstruction]

On the 50th or 79th day after the transplantation, the abdomen was opened again by midline abdominal incision. After the abdomen was opened, the greater omentum and intestine were manually moved to identify the transplanted tissue around the aorta. Furthermore, after the identification, the right renal artery was ligated, furosemide (20 mg) and indigocarmine (20 mg) were administered by intravenous injection, and about 100 mL of an extracellular fluid (normal saline, glucose, and VITAMEDIN (registered trademark)) was administered through an intravenous cannula. As a result of monitoring for 15 minutes or longer, urine production from the transplanted tissue was confirmed. Thereafter, the transplanted tissue was dissected together with the surrounding tissue and evaluated to finish the experiment.

### [Immunosuppression, anti-inflammatory, and supportive therapy]

Table 1 shows the details of immunosuppressive therapy. Regarding the start day, the day of transplantation is represented by (0), and the preceding days are represented by the minus sign (-). Specifically, in the therapy, tacrolimus (0.02 mg/kg im × 2/day; day -9 from start day), abatacept (50 mg/kg iv × 1/ week; day 7 from start day), and mycophenolate mofetil (40 mg/kg po × 2/day; day -5 from start day) were used. The administration of methylprednisolone (10 mg/kg iv) was also carried out from day 0 from the start day, which was changed to oral administration (5 mg/kg) from the next day. The dose was gradually reduced to 4, 3, 2, 1, 0.5, and 0.25 mg/kg every 5 days, and then the dose of 0.25 mg/kg was maintained until the time of autopsy. Furthermore, thymoglobulin (10 mg/kg iv on day 3), rituximab (10 mg/kg iv on day 2), tocilizumab (10 mg/kg sc on day -1, 7, and 14), and etanercept (0.5 mg/kg sc on day 0, 3, 6, and 10) were administered. The concentrations of tacrolimus and mycophenolate mofetil in blood were measured twice a week, and the dose was appropriately adjusted based on the target trough level (TAC trough level: 15 to 20 ng/mL on day -9 to 2; 9 to 12 ng/mL from day 3, MMF trough level: 4 to 6 µg/mL).

**[Table 1]**

| **Agent** | | **dose(duration)** |
|---|---|---|
| **Induction** | | |
| | Thymoglobulin(ATG) (Sanofi) | 10mg/kg IV (day -3) |
| | Anti-CD20mAb(rituximab) (Zenyaku Kogyo) | 10mg/kg IV (day -2) |

| **Maintenance** | | |
|---|---|---|
| | Abatacept (BMS) | 50mg/kg IV (every 7 days) |
| | Tacrolimus (Astellas) | 0.06-0.01 mg/kg x2/day IM (target trough 9-12ng/ml (day-9 to 2 target trough 15-20ng/ml)) |
| | Mycophenolate mofetil (Pfizer) | 10-50mg/kg x2/day PO (target trough 4-6µg/ml) |
| | Metylprednisolone (Pfizer) | 5mg/kg/day PO tapering to 0.5mg/kg/day |

| **Anti-inflammatory** | | |
|---|---|---|
| | Tocilizumab(IL-6R blockade) (Chugai) | 10mg/kg SC (days -1,7,14 and every 2weeks) |
| | Etanercept(TNF- α antagonist) (Pfizer) | 0.5mg/kg SC (days 0,3,6,10) |

| **Adjunctive** | | |
|---|---|---|
| | Aspirin (Bayer) | 40mg/kg/day PO |
| | Low molecular weight heparin (Kaken) | 700IU/day SC |
| | Erythropoietin (Kyowa Kirin) | 2000-4000IU SC (2 times weekly) |
| | Famotidine (LTL) | 0.25mg/kg PO x2/day |
| | Valganciclovir (Mitsubishi) | 15mg/kg PO |
| | Benzylpenicillin (Meiji Seika) | 95000IU/day IM |

### [Monitoring of recipient monkeys]

The daily dietary status and activity of the recipient monkets were monitored, and support was provided through supplementation as needed. Furthemore, the systemic condition of the monkeys was evaluated by the measurement of body weight, body temperature, and SpO2 as well as blood sampling conducted twice a week. In addition to TAC and MMF, RBC, Hb, Ht (MCV, MCH, and MCHC), RET, WBC (neutrophil, lymphocyte, monocyte, eosinophil, and basophil), Plt, PT, APTT, Fbg, TP, Alb, T-Bil, LDH, AST, ALT, ALP, γGTP CK, BUN, Cr, Na, K, Cl, Ca, P, Glu, T-Cho, TG, and CRP were also measured by blood test.

### [Histological and immunohistochemical evaluation]

The recovered specimens were fixed with 4% paraformaldehyde and embedded in paraffin. Next, the samples were isolated as sections with a thickness of 4 µm and stained using hematoxylin and eosin (HE), periodic acid-Schiff, and Masson's Trichrome staining.

In immunohistochemistry, serial sections with a thickness of 4 µm from the formalin-fixed specimens were deparaffinized and hydrated using ethanol in deionized water with graded concentrations.

The tissue section for CD3 staining was treated in a 0.01 M sodium citrate buffer for 10 seconds by using an autoclave (preheating the buffer for 30 seconds), and antigens were recovered. After the recovery of antigens, all the sections were gently washed with PBS. Endogenous peroxidase blocking was performed for 20 minutes by using 3% hydrogen peroxide. After being washed with PBS, all the sections were blocked with 5% skim milk for 30 minutes. Then, all the sections were incubated at 4°C overnight together with an anti-CD3 antibody (MA5-12577, Invitrogen, Carlsbad, CA, USA). After being washed with PBS, the sections were incubated for 30 minutes together with HISTOFINESIMPLESTAIN (registered trademark) (424151, NICHIREI CORPORATION., Japan). Diaminobenzidine was used as a chromogen, and hematoxylin was used as a counterstain.

### [Experiment 1: Immunological advantage of embryonic kidney on rejection reaction against different species]

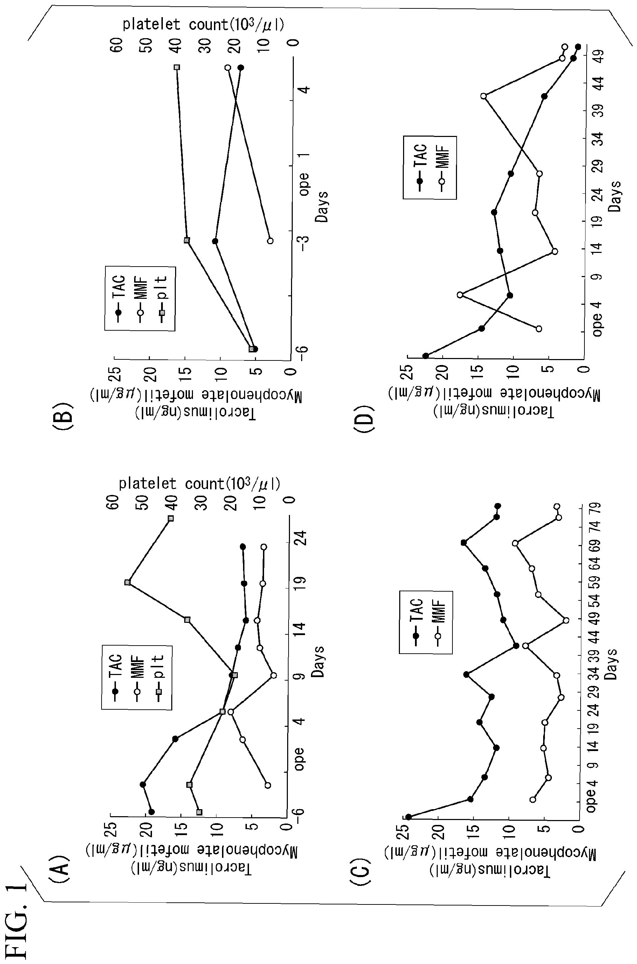

The dose of the immunosuppressants was increased or decreased based on the systemic condition and the results of blood biochemical test. The obtained pharmacokinetics are shown in FIGS. 1(A) and 1(B) (the results of Experiment 2 are also shown in FIGS. 1(C) and 1(D)). After the start of administration of immunosuppressants, a temporary increase in liver enzymes was confirmed in all cases, and the absorption of MMF was confirmed. Postoperative anemia was also observed.

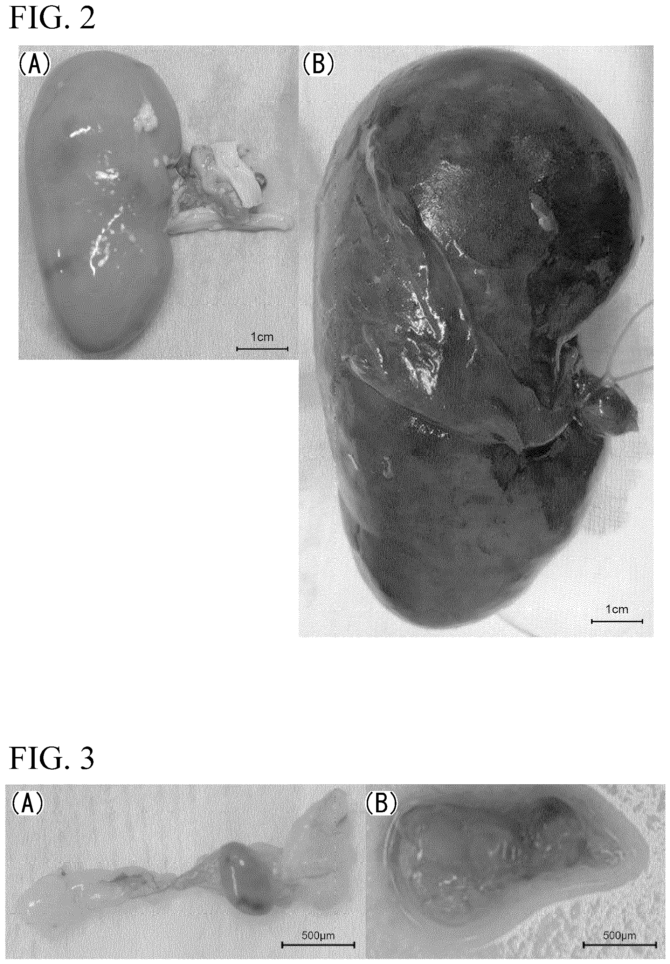

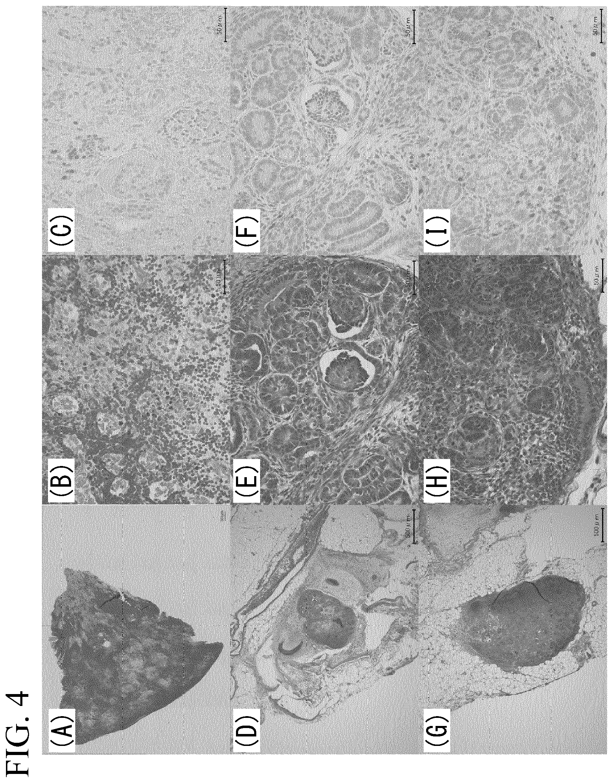

Because the symptoms suddenly aggravated on the 7th day after surgery, one case (corresponding to FIG. 1(B)) where the neonatal pig kidney and the embryonic cloaca were transplanted was invalidated. Other cases where the neonatal pig kidney and embryonic cloacae were transplanted were relatively stable. However, due to the deterioration of condition and platelet migration, the abdomen was opened again on the 13th day after transplantation, the pig kidney forming new blood vessels and the transplanted cloacae were extracted at the same time, and the cloacae were subjected to biopsy. The extracted transplanted kidney significantly swelled (weight 21 to 124 g, maximum diameter 5.2 to 10 cm) compared to the kidney at the time of transplant, and turned dark red. In contrast, the cloacae looked preserved (see FIGS. 2 and 3). In the transplanted kidney, the pathological evaluation revealed that the tissue was substantially disrupted, and a large amount of polynuclear cells and CD3-positive cells deeply stained by HE staining were observed (see FIGS. 4(A) to 4(C)).

On the other hand, the embryonic kidney (cloaca) recovered simultaneously with the aforementioned transplanted kidney retaining substantially all tissue structures, such as glomeruli and renal tubules. Furthermore, nuclear pleomorphism and infiltration of CD3-positive cells were not observed (see Figs, 4(D) to 4(F)). After the abdomen was closed again, immunosuppressant administration was continued to the same monkeys. After the transplanted pig kidney was removed, the monkeys recovered and remained relatively stable until the day of sacrifice, 27 days after the transplantation. Two recovered embryonic kidneys are shown in FIG. 2. On the 13th day after transplant, the tissue structure was found to be preserved, but infiltration of CD3-positive cells was observed in some cases (see Figs, 4(G) to 4(I)).

### [Experiment 2: Growth of embryonic cloaca by administration of FDA-approved immunosuppressants]

On the 33rd day after tissue transplantation, which was after about 4 weeks, the bladder grown from the transplanted embryonic cloaca was connected to the recipient by urinary tract anastomosis. Initially, the transplanted tissue was planned to be evaluated as a standard 6 weeks after the urinary tract anastomosis. However, because the condition of one case gradually deteriorated after the anastomosis, the transplanted tissue was removed 50 days after transplantation, and then the animal was euthanized. Other cases were relatively stable. Therefore, their tissues were recovered 46 days after the anastomosis (79 days after transplantation) as scheduled.

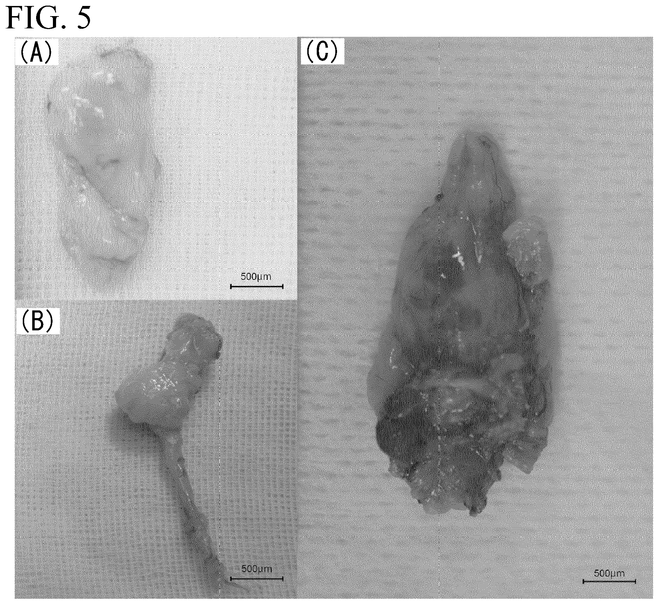

The major axis of the largest embryonic kidney recovered from the greater omentum 33 days after transplantation was about 800 µm (see FIG. 5(A)). Tissue structures such as glomeruli and renal tubules were substantially preserved as in Experiment 1, and no cellular infiltration was observed. The space of the Bowman's capsule (also called glomerular capsule) was found to be slightly enlarged by visual examination, and it was confirmed that urine was produced and the kidney was functioning.

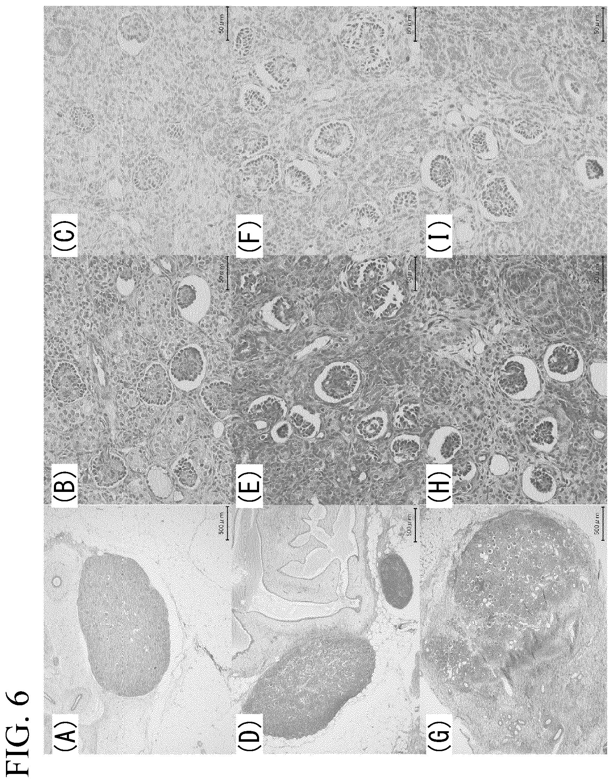

The major axis of the largest embryonic kidney recovered from the paraaortic region 50 days after transplantation was about 1.5 mm. Furthermore, the major axis of the largest embryonic kidney recovered from the paraaortic region 79 days after transplantation was about 2 mm (see FIGS. 5(B) and 5(C)). In histopathological evaluation, a significant change such as cellular infiltration was not observed in the embryonic kidney on the 33rd, 50th, and 79th days after transplantation (see FIG. 6).

The present example directly compares the immunogenicity of neonatal kidneys and embryonic kidneys in xenotransplantation between genetically unmodified pigs and monkeys, has succeeded in long-term growth of the embryonic kidneys (cloacae) after urinary tract anastomosis, and presents experimental results of observing the progress for the first time. These experimental results have revealed that the embryonic kidney functions more effectively than the neonatal kidney in xenotransplantation, and can achieve long-term survival.

In a case where the kidney of a mature pig is transplanted to a monkey, it is difficult to achieve long-term survival even though the pig is a genetically modified pig receiving only FDA-approved immunosuppressants. In the present invention, the transplantation of a neonatal pig kidney led to the deterioration of condition of one recipient monkey. The strong rejection reaction against the transplanted kidney was consistent with the results of previous reports. However, inflammatory cellular infiltration and tissue disruption that are typically seen in the rejection reaction in the neonatal pig kidney were substantially not observed in the embryonic kidney in the same period (renal anlage which is a cloaca).

In the case of the pig embryonic kidney, it has been confirmed that selecting an appropriate duration, such as 4 weeks, enables the embryonic kidney to mature into urine-producing renal tissue without differentiating into other tissues. Furthermore, the blood vessels of a xenogeneic recipient flow into the embryonic kidney in the process of transplantation, which suppresses the expression of donor antigens in the development stage of the kidney. These results indicate that the rejection reaction of the cloaca is less likely to occur in xenotransplantation. However, in Experiment 1 in which the neonatal kidney and the embryonic kidney were transplanted at the same time, inflammatory cellular infiltration was observed in the embryonic kidney even though the progress was shorter than in Experiment 2.

Accordingly, in a case where the elements that induce a strong inflammatory response are present as in the neonatal kidney, inflammation is likely to spread in the embryonic kidney that is considered to have low immunogenicity. In addition, natural antibodies such as anti-pig IgE/A antibodies are likely to be involved in the immune response of the recipient monkey, but are not considered to significantly affect the development of the embryonic kidney.

As confirmed in Experiment 2, in the transplantation of only the embryonic kidney (cloaca), reducing the type and dose of the immunosuppressants is expected to result in long-term survival. In order to develop the embryonic kidney into the more mature kidney of the recipient, not only the control of the rejection reaction but also the establishment of a urinary excretion system are necessary, which were accomplished by the present example.

In conclusion, the present example has succeeded in transplantation and long-term survival of embryonic kidneys by using the pig-monkey preclinical model without using genetically modified animals. This result is an important finding in developing a chimeric embryonic kidney into which human-derived renal progenitor cells are injected to achieve a humanized kidney.

### [Industrial Applicability]

According to the present invention, it is possible to provide a transplant material and a kidney transplant kit that are capable of suppressing the rejection reaction of a recipient when being transplanted into primates and accomplishing long-term survival.

## Claims

1. A transplant material used for kidney xenotransplantation to primates, the transplant material comprising:
a kidney anlage with bladder derived from a pig fetus devoid of blood vessels.

2. The transplant material according to Claim 1,
wherein the kidney anlage with bladder is derived from a pig fetus in week 4 to 6 of an embryonic period.

3. The transplant material according to Claim 1 or 2,
wherein the kidney anlage with bladder is derived from a wild-type pig fetus.

4. The transplant material according to any one of Claims 1 to 3,
wherein the kidney anlage with bladder further comprises human-derived nephron progenitor cells.

5. A kidney transplant kit, comprising:
the transplant material according to any one of Claims 1 to 4; and
a combination of drugs used based on an immunosuppressive protocol.

6. The kidney transplant kit according to Claim 5,
wherein the immunosuppressive protocol includes (1) administering at least one immunosuppressant selected from thymoglobulin and an anti-CD20 antibody, and (2) administering at least one immunosuppressant selected from a CD28-mediated co-stimulatory signal inhibitor, a calcineurin inhibitor, an inosine monophosphate dehydrogenase inhibitor, a steroid, an anti-CD25 antibody, and an anti-CD40 antibody.

7. The kidney transplant kit according to Claim 6,
wherein the immunosuppressive protocol further includes (3) administering at least one anti-inflammatory agent selected from an anti-IL-6 receptor antibody and a TNF-α inhibitor, and/or (4) administering at least one adjunctive agent selected from a platelet aggregation inhibitor, an anticoagulant, an antibiotic, a hematopoietic agent, a gastric acid suppressant, and an antiviral agent.

8. The kidney transplant kit according to any one of Claims 5 to 7,
wherein the immunosuppressive protocol includes
(1) independently administering thymoglobulin and an anti-CD20 antibody 2 or 3 days before transplantation,
(2) administering a CD28-mediated co-stimulatory signal inhibitor every 7 days after the transplantation, or immediately after the transplantation and every 7 days,
(3) continuously administering a calcineurin inhibitor from 9 days before the transplantation,
(4) continuously administering an inosine monophosphate dehydrogenase inhibitor from 5 days before the transplantation,
(5) continuously administering a steroid from the day of the transplantation,
(6) administering an anti-IL-6 receptor antibody 1 to 3 times on the day before the transplantation and about every 7 days after the transplantation,
(7) administering a TNF-α inhibitor every 3 or 4 days immediately after the transplantation, and
(8) administering at least one agent selected from a platelet aggregation inhibitor, an anticoagulant, an antibiotic, a hematopoietic agent, a gastric acid suppressant, and an antiviral agent as an adjunctive agent.

9. The kidney transplant kit according to any one of Claims 5 to 7,
wherein the immunosuppressive protocol includes
(1) administering an anti-CD20 antibody about 2 to 3 weeks before transplantation,
(2) continuously administering an inosine monophosphate dehydrogenase inhibitor from about 2 weeks before the transplantation,
(3) administering an anti-CD25 antibody on the day of the transplantation and 4^{th} day after the transplantation,
(4) continuously administering a calcineurin inhibitor and a steroid from about 1 week before the transplantation, and
(5) administering at least one agent selected from a platelet aggregation inhibitor, an anticoagulant, an antibiotic, a hematopoietic agent, a gastric acid suppressant, and an antiviral agent as an adjunctive agent.
